Europäisches Patentamt

European Patent Office

Office européen des brevets

(19)

(11) Publication number: 0 394 827
A1

# EUROPEAN PATENT APPLICATION

(21) Application number: 90107393.2

(22) Date of filing: 19.04.90

(51) Int. Cl.5: C12N 15/62, C12N 15/12,
C12N 15/13, C12N 15/85,
C12N 1/11, C12N 5/10,
A61K 37/02, C12P 21/00

The microorganism(s) has (have) been deposited with der Deutsche Sammlung von Mikroorganismen und Zellculturen GmbH under number(s) DSM 5173, 5174, 5314, 5315 und 5523.

(30) Priority: 26.04.89 EP 89107572
23.09.89 EP 89117606

(43) Date of publication of application:
31.10.90 Bulletin 90/44

(84) Designated Contracting States:
AT BE CH DE DK ES FR GB GR IT LI LU NL SE

(71) Applicant: F. HOFFMANN-LA ROCHE AG
Postfach 3255
CH-4002 Basel(CH)

(72) Inventor: Karjalainen, Klaus, Dr.
12 Andreasplatz
CH-4051 Basle(CH)
Inventor: Traunecker, André
11 rue du moulin
F-68450 Wolschwiller(FR)

(74) Representative: Mezger, Wolfgang, Dr. et al
Grenzacherstrasse 124 Postfach 3255
CH-4002 Basel(CH)

(54) Chimaeric CD4-immunoglobulin polypeptides.

(57) The present invention relates to a cloned DNA comprising a combination of two pieces of DNA whereby one of said pieces is coding for the first two domains or for parts thereof of the human CD4-molecule and the other is coding for all domains except the first of the constant regions of the heavy chain of a mammalian immunoglobulin, to vectors containing such DNA, to prokaryotic or eukaryotic host cells transformed with such vectors, to polypeptides encoded by such DNA, to a method for the production of such polypeptides, to pharmaceutical compositions containing such polypeptides and to the use of such polypeptides for the preparation of pharmaceutical compositions, especially such compositions for the treatment of AIDS.

EP 0 394 827 A1

## Chimaeric CD4-Immunoglobulin Polypeptides

Soluble CD4 provides the optimal specificity for neutralization of the Human Immunodeficiency Virus (HIV) for several reasons. Firstly, the strength of the interaction between CD4 and the HIV-associated ligand gp120 is very high, viz. in the order of $10^{-9}$M (Smith, D.H. et al., Science 238, 1704-1707, 1987; Lasky, L. et al., Cell 50, 975-985, 1987). Secondly, the genetic variants of HIV which emerge frequently and may represent an "escape" mechanism from the immune surveillance (Klatzmann and Gluckman, Immunol. Today 7, 291-296, [1986]) must retain their CD4 binding properties in order to maintain their infection cycle. Thirdly, the immunity against the HIV-envelope protein gp120 acquired during the infection can have deleterious effects on the immune system: free gp120, which is shed from the virus (Schneider, J. et al., J. Gen. Virol. 67, 2533-2539, [1986]) can be trapped specifically on CD4-positive cells. In this way these non-infected cells can become targets of various forms of anti-gp120 immune attacks (Lyerly, H.K. et al., PNAS USA 84, 4601-4605, [1987]; Weinhold, K.J. et al., Lancet, 902-905, [1988]; Lanzavecchia, A. et al., Nature 334, 530-534, [1988]; Siliciano, R.F. et al., Cell 54, 561-575 [1988]). The passive immunity based on CD4 specificity avoids this bystander destruction because it can distinguish gp120 molecules bound on the cell surface-associated CD4 receptor from those produced by virally infected cells. The preparation of soluble CD4 molecules has been reported for example by Smith, D.H. et al., Science 238, 1704-1707, (1987); Fisher, R.A. et al, Nature 331, 76-78, (1988); Hussey, R.E. et al, Nature 331, 78-81 (1988); Deen, K.C et al., Nature 331, 82-84 (1988); Traunecker, A. et al, Nature 331, 84-86 (1988) and by researchers from the Columbia University (see WO 88/01304). In addition, Traunecker et al. (Nature 331, 84-86, 1988) demonstrated that the first two domains of CD4 when fused to the constant domain of a immunoglobulin light chain were sufficient to inhibit HIV infection in vitro.

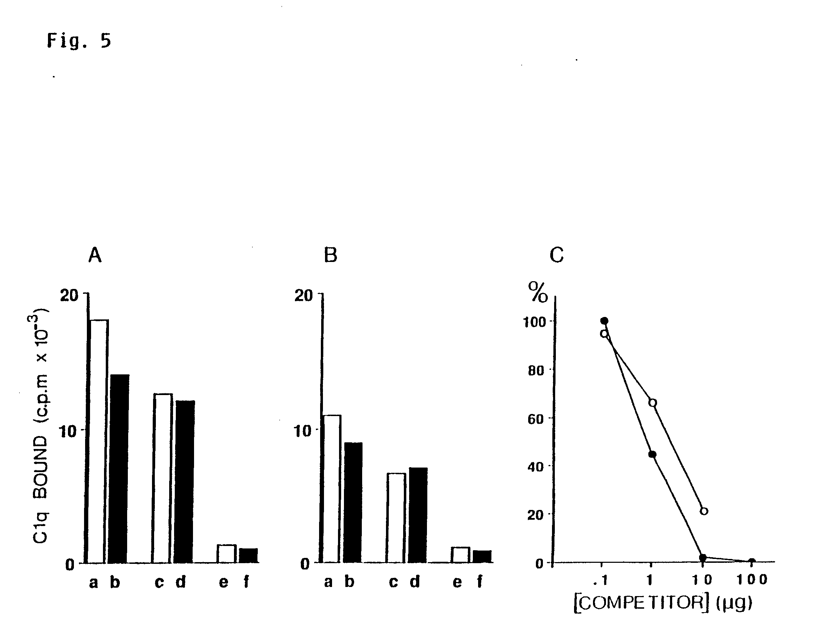

Combining such soluble CD4-molecules with different immunoglobulin heavy chain molecules leads to so called chimaeric CD4-immunoglobulin polypeptides which could have the following advantages in addition to those already mentioned for the soluble CD4-molecules. The immunoglobulin parts may confer their great stability to the rest of the chimaeric polypeptides, thereby possibly increasing their half-life in vivo, which is, e.g., for human IgG 23 days and for IgM 5 days (Waldman et al. in "Immunoglobulins" ed. by E. Merler, 1970, pp. 33-48. National Academy of Science, Washington, DC). In addition, the constant regions of both the $\gamma$(IgG) and $\mu$(IgM) chains could fix complement, making neutralization or killing of HIV viruses or virus producing cells much more efficient (see Yarchoun et al. in Scientific American, October 1988). Increased half-life in vivo has been shown for chimaeric polypeptides consisting of the first two or all four CD4-domains and all three domains of the human IgG₁ heavy chain constant region by Capon, D.J., et al, in Nature 337, 525-531 (1989). Binding of complement to these specific chimaeric polypeptides, was not observed. However, binding of complement to the chimaeric polypeptides of the present invention could be shown [for details see Example 5 and Figure 5]. In addition these polypeptides bind to Fc-receptors [Example 6 and Figure 5] and thereby could attract lymphocytes capable of mediating antibody dependent cell cytotoxicity (ADCC). Binding to Fc-receptors was also shown by Capon. D.J., et al. (see above) for their chimaeric polypeptides. All these effects are an advantage of chimaeric CD4-immunoglobulin heavy chain polypeptides over chimaeric CD4-immunoglobulin light chain polypeptides described by Traunecker et al. in Nature 331, 84-86 (1988) and in Immunology Today 10, 29-32 (1989) because such effects are attributed to the heavy chain and not the light chain part of the chimaeric polypeptides.

Furthermore, some of the polypeptides of the present invention as defined below form defined oligomers containing several gp120 binding sites in one polymeric structure, thereby increasing the HIV-inhibitory effect in a HIV-dependent syncytium assay by a factor of 1000, as found for the chimaeric CD4-IgM heavy chain polypeptides when compared with, e.g., the chimaeric polypeptide described by Traunecker et al. in Nature 331, 84-86 (1988).

The present invention relates among others to a cloned DNA comprising a combination of two pieces of DNA wherein one of said pieces is coding for the first two domains or for parts thereof of the human CD4-molecule and the other is coding for all domains except the first of the constant region of the heavy chain of a mammalian immunoglobulin. Under parts of the first two domains of the human CD4-molecule the following possibilities are understood:

(a) the complete first domain in combination with parts of the second domain, or

(b) the complete second domain in combination with parts of the first domain, or

(c) a combination of parts of either domain.

A preferred embodiment of the present invention is a cloned DNA wherein one of said pieces is coding for the first two domains of the human CD4-molecule.

Another preferred embodiment is a cloned DNA wherein one of said pieces is as characterized above and the other of said pieces of DNA is coding for all domains except the first of the constant region of the heavy chain of (a) a mammalian IgM, (b) a mammalian IgG, or (c) a mammalian IgA.

Another preferred embodiment of the present invention is a cloned DNA as defined above wherein the mammalian immunoglobulin is mouse or human immunoglobulin.

Especially preferred embodiments of the present invention are cloned DNAs as defined above wherein the CD4 coding part is as above and the other of said pieces of DNA is coding for all domains except the first of the constant region of the heavy chain of (a) mouse Ig $\gamma$2a or (b) mouse Ig $\mu$ or (c) human Ig $\mu$ or (d) human Ig $\gamma$1 or (e) human Ig $\gamma$3 or (f) human Ig $\alpha$1 or (g) human Ig $\alpha$2.

The present invention relates further to vectors containing a DNA as defined above, preferably to shuttle vectors capable of replicating in prokaryotic and eukaryotic host cells and more preferably to shuttle vectors capable of expressing the protein encoded by the integrated DNA in appropriate host cells.

Another object of the present invention are prokaryotic and eukaryotic host cells transformed with a vector as defined above as well as a method for the production of polypeptides encoded by the DNAs as defined above by culturing in a suitable medium such transformed host cells.

Another embodiment of the present invention are the polypeptides themself encoded by the DNAs as defined above. Such polypeptides are of chimaeric nature comprising the first two domains of the human CD4-molecule or fragments thereof and all domains except the first of the constant region of the heavy chain of a mammalian immunoglobulin (Ig). Preferred polypeptides are those which besides the Ig part comprise the first two domains of the human CD4-molecule. In an especially preferred embodiment the CD4-part of these polypeptides comprises the amino acid sequence:

```
K K V V L G K K G D T V E L T C T A S Q K K S I Q F H W K N
S N Q I K I L G N Q S F L T K G P S K L N D R A D S R R S L
W D Q G N F P L I I K N L K I E D S D T Y I C E V E D Q K E
E V Q L L V F G L T A N S D T H L L Q G Q S L T L T L E S P
P G S S P S V Q C R S P R G K N I Q G G K T L S V S Q L E L
Q D S G T W T C T V L Q N Q K K V E F K I D I V V L
```

Other preferred polypeptides of the present invention are those encoded by the DNAs specifically mentioned above.

Pharmaceutical compositions containing at least one of said polypeptides and a therapeutically acceptable carrier material as well as their preparation are another embodiment of the present invention.

Finally, the present invention relates also to the use of said polypeptides, viz. for the preparation of pharmaceutical compositions and for the treatment of various diseases, especially for the treatment of AIDS.

The invention will be better understood on the basis of the following detailed description when considered in connection with the accompanying drawings, wherein:

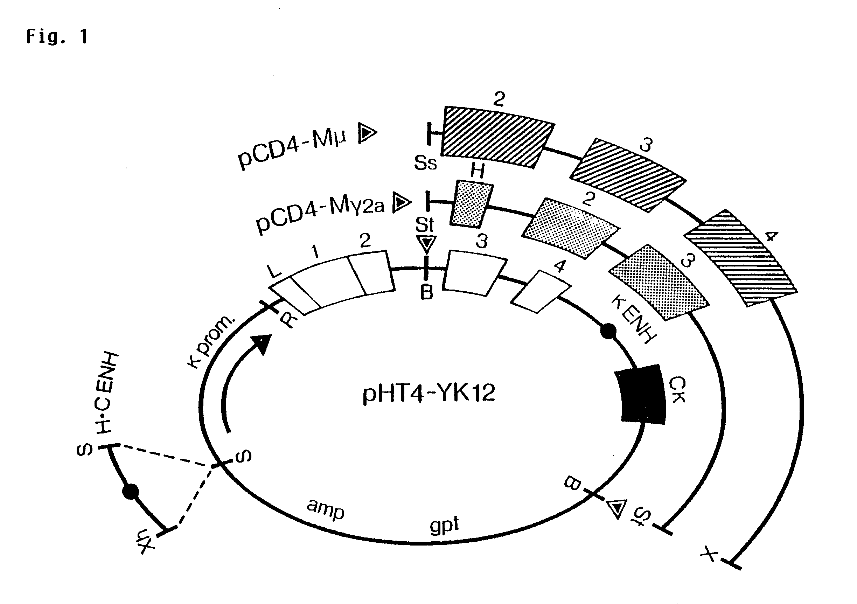

Figure 1 shows the plasmids pHT4-YK12, pCD4-M$\mu$ and pCD4-M$\gamma$2a. Boxes refer to pieces of cDNA equivalent to specific parts of exons [see Example 1] or exons themselves used for the construction of the plasmids wherein the white boxes refer to DNA coding for the leader region (L) and all four (1,2,3,4) extracellular domains of the human CD4-molecule. The black box refers to the exon coding for the immunoglobulin $x$ light-chain constant region (c$x$). The dotted boxes refer to the exons coding for the hinge (H) and the second and third domains of the mouse IgG$_{2a}$ constant regions (C2, C3) respectively. The striped boxes refer to the exons coding for the second, third and fourth domains of the mouse IgM constant regions respectively. Further abbreviations refer to: B = BamHI restriction site, R = EcoRI restriction site, S = sall restriction site, Ss = SstI restriction site, St = StuI restriction site, X = XbaI restriction site, Xh = XhoI restriction site, amp = ampicillin resistance gene. gpt = E. coli xanthine-guanine phosphoribosyltrans-ferase gene, $x$ prom = Ig$x$ promotor, $x$ENH = Ig$x$ enhancer, H•CENH = Ig heavy chain enhancer.

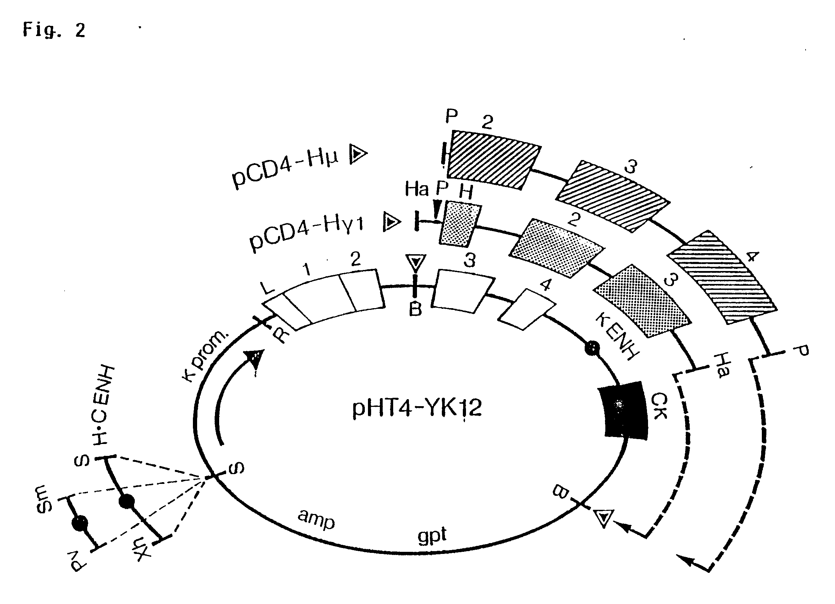

Figure 2 shows plasmids pHT4-YK12 (see Figure 1), pCD4-H$\mu$ and pCD4-H$\gamma$l. In these latter plasmids the boxes represent the human IgG and IgM regions corresponding to the mouse exons of Fig. 1. "P" refers to a PstI restriction site and "Ha" to a HaeII restriction site, "Pv" to a PvuII restriction site and "Sm" to a SmaI restriction site. All other symbols and abbreviations have the same meaning as in Figure 1.

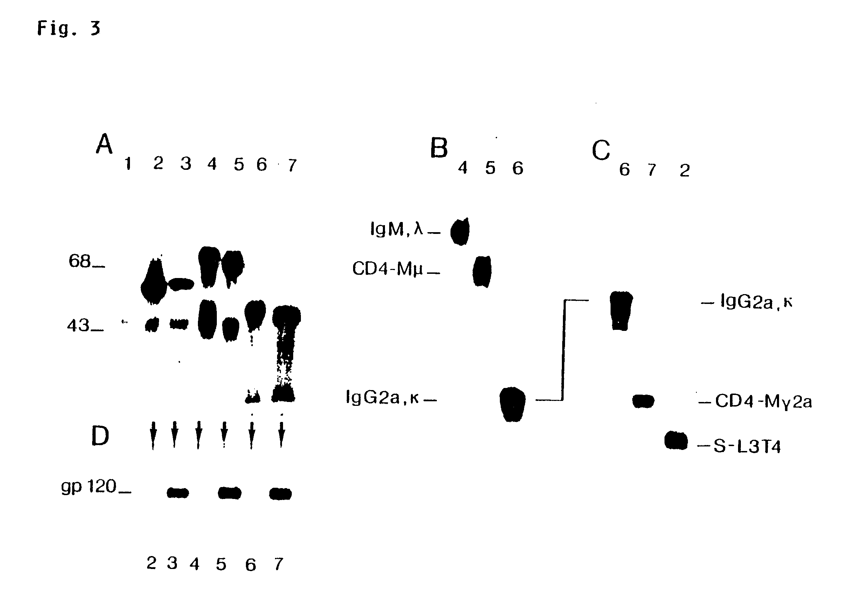

Figure 3 gives a Western blot analysis of the chimaeric polypeptides, "CD4-M$\mu$" and "CD4-M$\gamma$2a" encoded by the cloned DNA incorporated into the corresponding plasmids pCD4-M$\mu$ and pCD4-M$\gamma$2a as given in Figure 1. Lanes A show such a blot of a 10% polyacrylamide gel (PAGE) run under reducing conditions of immunoprecipitated chimaeric-polypeptides or natural Ig molecules from cell culture supernatents or as in the case of (1) of an immunoprecipitated cell culture supernatant:

(1) X63-0: X63-0 myeloma cell line [identical with P3X63Ag8 cell line] secreting no Ig molecules

(2) L3T4: soluble mouse CD4-molecule secreted by a cell line described by Traunecker et al. in Immunol. Today 10, 29-32, (1989).

(3) HT4-Y1: X63-0 myeloma cell line transfected with pHT4-Y1 [Traunecker et al., Nature 331, 84-86 (1988)] secreting a chimaeric CD4-mouse Ig xlight chain polypeptide

(4) B1.8/IgM,λ: cell line [Reth, M. et al., Europ. J. Immunol. 8, 393 (1978)] secreting a mouse IgM

(5) CD4-Mμ: X63-0 myeloma cell line transfected with pCD4-Mμ secreting the chimaeric CD4-Mμ-polypeptide.

(6) UPC 10/IgG2a,K: UPC 10 cell line [Litton Bionetics, Kensington, Maryland, USA] secreting IgG2a.

(7) CD4-Mγ2a: X63-0 myeloma cell line transfected with pCD4-Mγ2a secreting the chimaeric CD4-Mγ2a polypeptide. Samples 1 to 3 were immunoprecipitated with rabbit anti-mouse K, samples 4 and 5 with rabbit anti-mouse IgM, samples 6 and 7 with protein-A. All antibodies and protein A were immobilized on Sepharose 4B (Pharmacia, Uppsala, Sweden). Western blot analysis was performed after 3 to 7% gradient PAGE(B) or 7% PAGE(C) run under non-reducing conditions. The results were visualized with a mixture of rabbit anti-mouse Igs (Nordic Labs, NL) in a first step followed by visualization with donkey $^{125}$I-labelled anti-rabbit Ig (Amersham, Aylesbury, GB). Standard molecular weight markers are shown (kd) on the left (A). The background bands in (A) (1 to 5) of about 45 kd are due to rabbit IgG released from the Sepharose when the samples were prepared for electrophoresis. Coimmunoprecipitation under identical conditions as in (A)-(C) but in the presence of metabolically labelled gp120 from a HIV I infected H9 cell culture supernatant followed by PAGE and fluorography is shown in (D).

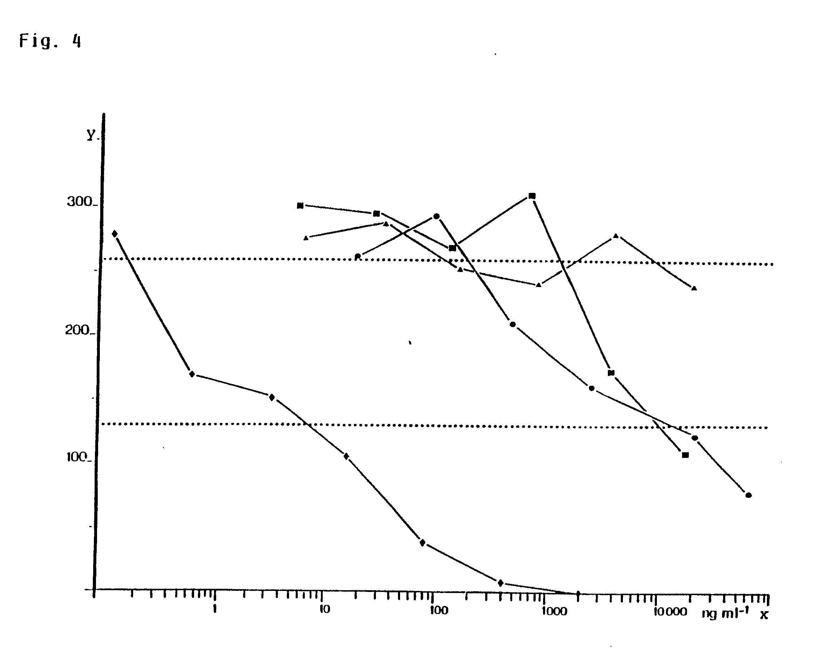

Figure 4 shows the degree of inhibition of syncytium formation with respect to the following recombinant proteins, whereby the concentrations of such proteins are given in a logarithmic scale on the X axis and the Y axis gives the numbers of syncytia scored in triplicate evaluations:

♦: CD4-Mμ = chimaeric protein encoded by the DNA of the plasmid pCD4-Mμ.

■: CD4-Mγ2a - chimaeric protein encoded by the DNA of the plasmid pCD4-Mγ2a

●: CD4-Mx - chimaeric protein encoded by the DNA of the plasmid pHT4-YKl2

▲: L3T4-Mx = soluble mouse CD4-molecule as described by Traunecker et al. (Immunol. Today 10, 29-32, [1989])

Figure 5 (A.B) shows the results of an assay of the binding of CD4-Mμ and CD4-Mγ2a to Clq, the first component of the complement cascade via the binding site of their heavy chain parts [Burton, D., Molecul. Immunol. 22, 161-206 (1985)] as determined according to Example 5. Microtiter plates were coated with purified CD4-Igs and natural Igs as controls at concentrations of 10 μg/ml (A) and 1μg/ml (B). The direct binding of $^{125}$I-labelled Clg is given in counts per minute (c.p.m). Lane a: CD4-Mγ2a; lane b: monoclonal antibody OKT3, which is a IgG2a, x (Ortho Diagnostics); lane c: CD4-Mμ: lane d: mouse IgM,x from TEPC 183 (Litton Bionetics); lane e: CD4-Mx [Traunecker et al., Nature 331, 84-86 (1988)]; lane f: Bovine Serum Albumin.

Figure 5(C) shows the results of the binding of chimaeric protein CD4-Mγ2a to Fcγ receptors on the mouse macrophage cell line J 774 A.1 (ATCC No. TIB 67). % of competition of $^{125}$I labelled CD4-Mγ2a is given in dependency of cold competitor [(O):CD4-Mγ2a, (●): cold IgG2a = monoclonal antibody OKT3] as determined according to Example 6.

The present invention relates also to a method for the production of polypeptides encoded by a DNA of the invention which method comprises culturing in a suitable medium a host cell transformed with a vector capable of expressing said polypeptide and isolating said polypeptide.

It is well known in the art that the cloned DNA of the invention can be obtained on the one hand in the form of a cDNA starting from an appropriate mRNA preparation as described for example by Maniatis, T. et al. in "Molecular Cloning" (1982), Cold Spring Harbour Laboratory, or more specifically for example by Maddon, J.P. in Cell 42 (1985), 93-104 for a cDNA encoding the human CD4-molecule. In the case that such a cDNA encodes more than the desired number of domains for the practice of the present invention said cDNA has to be cleaved on the basis of the knowledge of its sequence and at least the partial knowledge of the amino acid sequence of the desired domains by nuclease treatment with which a man skilled in the art is familiar.

On the other hand such cloned DNA can be obtained in the form of genomic DNA isolated from a genomic library by methods well known in the art and described for example by Maniatis, T. et al. in "Molecular Cloning" (see above). For example specific oligonucleotide probes could be prepared on the at least partial knowledge of the amino acid sequence encoded by the specific genomic DNA (exons) to be isolated or the at least partial knowledge of its nucleic acid sequence. Such sequence information can be obtained in principle from any sequence data base, for example, like Genbank (Intelligenetics, California,

4

USA), EMBL (Heidelberg, FRG), NBRF (Georgetown University, Medical Centre, Washington DC, USA) and Vecbase (University of Wisconsin, Biotechnology Centre, Madison, Wisconsin, USA) or more specifically for example for the human CD4-molecule from Maddon et al. (1985) in Cell 42, 93-104, for the mouse Ig $\mu$ from Arnheim, N. et at., Cell 22, 179-185 (1980), for the mouse Ig$\gamma$2a from Roeder, W. et al., PNAS 78, 474-478 (1981), for the human Ig $\mu$ from Rabbits, T.H. et al., Nucleic Acids Res. 9, 4509-4524 (1981), for the human Ig$\gamma$l from Ellison, J.W. et al, Nucleic Acids Res 10, 4071-4079 (1982), for the human Ig $\gamma$3 heavy chain gene from Huch, S. et al., Nucleic Acids Res. 14, 1779-1789 (1986) and for the human Ig$\alpha$1 and Ig$\alpha$2 heavy chain gene from Flanagan, J.G. et al., Cell 36, 681-688 (1984).

The genomic clones of the desired pieces of DNA can then be detected by the use of oligonucleotide probes designed as described above under specific hybridization conditions known to and eligible by a man skilled in the art.

Cloned pieces of DNA coding for the different human CD4- and immunoglobulin heavy chain domains obtained as described above can then be handeled and integrated into suitable vectors according to methods well known in the art and described for example by Maniatis et al. in "Molecular cloning" (see above). It is well understood and covered by the present invention that the DNA pieces coding for the chimaeric polypeptides may be of different origin, viz. partly of cDNA and partly of genomic origin.

Suitable vectors for the practice of the present invention comprise vectors which are known in the art and generally used for the replication of integrated DNA in prokaryotic or eukaryotic host cells, especially vectors which can be used for such purpose in both prokaryotic and eukaryotic host cells, called shuttle vectors and vectors for the expression of polypeptides in eukaryotic host cells. Especially preferred are shuttle vectors which can be used for the expression of polypeptides in eukaryotic host cells like, e.g., pSV2 derived vectors [described, for example, by German, C. in "DNA Cloning", Vol. II., ed. by Glover, D.M., IRL Press, Oxford, 1985] like pSV2-cat [ATCC No. 37 155], pSV2-dhfr [ATCC No. 37146], pSV2-neo [ATCC No. 37149] or pSV2-gpt [ATCC No. 37145]. pSV2-gpt derived vectors, especially vectors like PHT4-Y1, pCD4-M$\gamma$2a, pCD4-M$\mu$, pCD4-H$\mu$, pCD4-H$\gamma$1 and pCD4-H$\gamma$3 are preferred. Plasmids pCD4-M$\gamma$2a and pCD4-M$\mu$ have been deposited under the Budapest Treaty for patent purposes at the Deutsche Sammlung von Mikroorganismen und Zellkulturen GmbH (DSM) in Braunschweig, BRD, at the 26th of January 1989 under accession numbers DSM 5173 and DSM 5174 respectively, plasmids pCD4-H$\mu$ and pCD4-H$\gamma$1 at the 21st of April 1989 under accession numbers DSM 5315 and DSM 5314 respectively and plasmid pCD4-H$\gamma$3 at the 14th of September 1989 under accession number DSM 5523.

Prokaryotic or eukaryotic host cells can then be transformed by such vectors. Suitable prokaryotic host cells for such purpose are well-known to a person skilled in the art and include bacteria, for example, E. coli, especially E. coli K12 strains, e.g. M15 (described as DZ 291 by Villarejo et al. in J. Bacteriol. 120, 466-474 [1974]) and HB 101 [ATCC No. 33694].

Suitable eukaryotic host cells also well known to skilled art persons include cells of vertebrate, yeast or other origin with myeloma cell lines like, e.g., P3X63Ag8 [ATCC No. TIB9] and J558 [ATCC No. TIB6] being preferred. In accordance with the specific host cell to be used in the practice of the present invention suitable promotor-elements like for example the Ig heavy chain promotor or the Ig$x$-promotor (see Example 1) and if desired suitable enhancer elements like for example the Ig heavy chain enhancer or the Ig light chain enhancer (see Example 1) can be integrated into the plasmids used for the transformation of such host cells by methods well known in the art.

Transformation of the host cells by vectors as described above may be carried out by any conventional procedure or by the specific procedures as described in the following Examples.

Where the host cell is a prokaryote such as E. coli for example, competent cells which are capable of DNA uptake are prepared from cells harvested after exponential growth phase and subsequently treated according to the known CaCl$_2$-method. Transformation can also be performed after forming a protoplast of the host cell or by other methods known in the art.

Where the host used is a eukaryote the following transfection methods like calcium phosphate precipitation and the DEAE-Dextran method (see also C. Gorman in "DNA Cloning", Vol. II), conventional mechanical procedures such as microinjection, insertion of a plasmid encapsulated in red blood cell hosts or in liposomes, treatment of cells with agents such as lysophosphatidylcholin, use of virus vectors or protoplast fusion can be employed in the practice of the present invention, with protoplast fusion being a preferred method (see Example 2).

Transformed cells can then be selected and cultivated according to methods well known in the art. For example cells which have been transformed by a vector carrying a "neo" gene coding for a phosphotransferase can now be cultured in the presence of the drug G418 or in case of a vector carrying a "gpt" gene transformed cells can be grown in specific media containing xanthine and mycophenolic acid (Gorman in "DNA Cloning", Vol II, see above).

Chimaeric polypeptides of the present invention produced by transformed cells as stated above can be recovered by any appropriate method known in protein and peptide chemistry such as, for example, precipitation with ammonium sulfate, dialysis, ultrafiltration, gelfiltration or ion-exchange chromatography, gel electrophoresis, isoelectric focusing, affinity chromatography, like immunoaffinity chromatography, HPLC or the like.

The chimaeric polypeptides of the present invention can be characterized for example by Western blot analysis (see Figure 3 and Example 2), a technique with which a man skilled in the art is familiar, or by their ability to inhibit syncytium formation in assays known in the art employing cell lines like for example: H9, MOLT-4 (ATCC No. CRL 1582), JURKAT, JM or CEM (ATCC No. CCL 119; Chaffee, S. et al., J. Expt. Med. 168, 605-621 (1988)) [see also Popovic et al., Lancet (1984)ii, 1472; Nara, P.L., and Fischinger, P.J., Nature 332, 469-470 (1988)] or as being specifically described in Example 4 in which MOLT-4-or CEM cell lines can substitute for the MT-2 cells. Any HIV-1 virus which has the ability to lead to syncytium formation such as for example: HTLV-IIIB, obtainable from infected H9 cell lines (ATCC No. CRL 8543) by methods known in the art, or for example as described by Popovic et al., Science 224, 497-499 (1984) or LAV (CNCM No. I-232) can be used in such assays. Another way of characterization of the polypeptides of the invention is by their ability to bind to the HIV surface protein gp120 in assays known in the art or as specifically described in Example 3 and visualized by fluorography according to standard techniques (Figure 3) or by their ability to bind to Clq, the first component of the complement cascade in assays known in the art or as specifically described in Example 5 and presented in Figure 5 (A und B). This figure clearly demonstrates that CD4-M$_\gamma$2a and CD4-M$\mu$ bind Clq as efficiently as natural antibodies, whereas the control construct CD4-M$x$, containing a light chain constant domain, does not show any specific binding. Chimaeric polypeptides of the present invention can also be characterized by their ability to bind to Fc-receptors present on monocytes/macrophages and neutrophils due to the Fc portion of their IgG heavy chain part [Burton, D., Molecul. Immunol. 22, 161-206 (1985)] in assays known in the art or as specifically described in Example 6 and presented in Figure 5 (C). This figure clearly shows that CD4-M$_\gamma$2a binds to Fc receptors on J774A.1 cells to the same extent and in indistinguishable affinity as mouse IgG2a. Binding of chimaeric human CD4-H$_\gamma$ polypeptides to Fc receptors can be demonstrated for example with the human monocyte / macrophage cell line U937 (ATCC No. CRL 1593) according to the method as given in Example 6. Furthermore such chimaeric polypeptides can be characterized for example by an assay described by Pauwels et al. [J. Virol. Methods 16, 171-185 (1987)] or their ability to inhibit transmission of the virus by cell to cell contact.

In accordance with the present invention the novel chimaeric proteins may be used in the treatment of various diseases, especially in the treatment of AIDS. They may be administered in pharmaceutically acceptable forms, especially in an injectable form. Dosage and dose rates may parallel that currently being used in clinical applications of other known polypeptides of comparable structure and/or function. Pharmaceutical compositions may contain at least one chimaeric polypeptide of the present invention in association with pharmaceutically acceptable solid or liquid carrier materials. Any conventionally used carrier material can be utilized. Furthermore, the pharmaceutical compositions may contain other pharmaceutically active agents and be prepared by methods known in the art.

Having now generally described the invention, the same will become better understood by reference to the specific examples which are included herein for purpose of illustration only and are not intended to be limiting unless otherwise specified.

Example 1

Construction of Plasmids

Plasmid constructions were carried out using standard methodology as described by Maniatis et al., Molecular Cloning: A Laboratory Manual, Cold spring Harbor Laboratory, New York (1982).

The starting plasmid for all constructions is designated pHT4-Y1 and can be prepared as previously described by Traunecker, Lüke and Karjalainen in Nature 331, 84-86 (1988). This vector, carrying as selection markers the ampicillin resistance gene and the gpt gene (Mulligan and Berg, 1980, Science 209, 1422), encodes in addition a hybrid protein composed of the whole extracellular portion of the human CD4 receptor fused to the mouse immunoglobulin kappa light-chain constant region.

Vector pHT4-Y1 was modified by replacing the mouse Ig heavy chain promoter by the more efficient mouse Ig kappa promoter derived from the vector pKm 1 (Traunecker et al., 1986, Eur. J. Immunol. 16, 851-854). The kappa promoter was first subcloned as a BglII/SalI fragment into the vector pUC18 digested with BamHI and SalI, recovered as a 2.2 kb HindIII (3' recessive ends filled with the help of the Klenow fragment of E. coli DNA polymerase, called hereafter: blunt) / EcoRI fragment and subsequently inserted into XbaI (blunt) / EcoRI digested pHT4-Y1 to generate plasmid pHT4-YK12 (see Fig. 1).

This plasmid was then digested with BamHI, to remove the exons 3 and 4 of the CD4 gene and the exon encoding the mouse Ig kappa light chain constant region, the 3' recessive ends were filled with Klenow polymerase and dephosphorylated with calf intestine alkaline phosphatase to facilitate the insertion of the following gene fragments (blunt ended before ligation):
- a 3.5 kb SstI/XbaI DNA fragment containing the constant region exons C2, C3 and C4 of the mouse Ig $\mu$ heavy chain gene [Arnheim N. et al., Cell 22, 179-185 (1980)] resulting in the preliminary plasmid pCD4-M$\mu$*.
- a 3.0 kb StuI DNA fragment containing the Hinge and the C2 and C3 constant region exons of the mouse Ig $\gamma$2a heavy chain gene [Roeder, W. et al., PNAS 78, 474-479 (1981)] resulting in the preliminary plasmid pCD4-M$\gamma$2a*.
- a HaeII DNA fragment (made blunt with T4 DNA polymerase) containing the Hinge and the C2 and C3 constant region exons of the human Ig$\gamma$1 heavy chain gene [Ellison, J.W. et al., Nucleic Acids Res. 10, 4071-4079 (1982)] resulting in the preliminary plasmid pCD4-H$\gamma$1*.
- a DNA fragment containing the constant region exons C2, C3 and C4 of the human Ig $\mu$ heavy chain gene [Rabbits, T.H. et al., Nucleic Acids Res. 9, 4509-4524 (1981)]. This DNA fragment was constructed as follows: first, the HaeII fragment of the human Ig $\gamma$1 heavy chain gene was made blunt ended as described and inserted into a blunt ended SalI site of pUC19 in an orientation positioning the Hinge exon towards the BamHI site. This intermediate construct was then digested with PstI to remove the 3 $\gamma$1 exons except for the splice acceptor site of the Hinge exon flanking the PstI site in the gene (the second PstI site is in the pUC19 polylinker), followed by the insertion of the PstI fragment of the human $\mu$ gene containing exons C2, C3 and C4 (Rabbits et al., see above). This final gene construct was then recovered by BamHI and HindIII digestion (both sites in the pUC19 polylinker), followed by Klenow treatment and insertion into vector pHT4-YK12 as described above resulting in the preliminary plasmid pCD4-H$\mu$*.

The preliminary plasmids (pCD4-M$\mu$*, pCD4-M$\gamma$2a*, pCD4-H$\gamma$1*) were completed by inserting a XhoI-SalI fragment which contains the murine $\mu$ heavy chain gene enhancer into the unique SalI site 5' to the promoter (see Figures 1 and 2) and the preliminary plasmid pCD4-H$\mu$* was completed by inserting the murine $\mu$ "core" enhancer as a SmaI-PvuII fragment into the blunt-ended SalI site 5' to the promotor (Figure 2), resulting in the final plasmids pCD4-M$\mu$, pCD4-M$\gamma$2a, pCD4-H$\gamma$1 and pCD4-H$\mu$. Originally the enhancer fragment XbaI-EcoRI described by Gillies et al. (Cell 33, 717-728, 1983) was inserted, via an EcoRI-linker on XbaI, into the EcoRI site of a Bluescript vector (Stratagene, La Jolla, USA) in an orientation positioning the XbaI(EcoRI) site next to the EcoRV site. Into the unique SstI site of the vector an adaptor was inserted which contained a XhoI site. Finally, the enhancer fragment was recovered with XhoI-SalI for constructs pCD4-M$\mu$, pCD4-M$\gamma$2a and pCD4-H$\gamma$1 or with SmaI-PvuII for construct pCD4-H$\mu$.

For the construction of pCD4-H$\gamma$3 plasmid PHT4-YK12 (see Fig. 1) was digested with BamHI to remove the exons 3 and 4 of the CD4 gene and the exon encoding the mouse Ig kappa light chain constant region, and dephosphorylated with calf intestine alkaline phosphatase to facilitate the insertion of a 6.0 kb BglII DNA fragment containing the hinge and the C2 and C3 constant region exons of the human Ig$\gamma$3 heavy chain gene [Huch, S. et al., Nucleic Acids Res. 14, 1779-1789 (1986)] resulting in the preliminary plasmid pCD4-H$\gamma$3 . The preliminary plasmid pCD4-H$\gamma$3* was completed by inserting the murine $\mu$ "cae" enhancer as described above for plasmid pCD4-H$\mu$.


Example 2


Expression of the CD4-M$\mu$ and CD4-M$\gamma$2a encoding genes


Maintenance of animal cells and cell-lines, cloning-methodology, selection procedures and expansion of cloned cells were carried out using standard methodology as described by Freshney, R.I. in "Culture of animal cells: A manual of basic technique, Alan R. Liss, Inc., New York (1983).

Mouse myeloma cells P3X63Ag8 (ATCC No. TIB9) or J558 (ATCC No. TIB6) were stably transfected with the plasmids pCD4-Mµ or pCD4-Mγ2a by protoplast fusion as described by Oi et al. (Proc. Natl. Acad. Sci. USA 80, 825-829, 1983). Transfectants were selected by including into the basic medium (Dulbecco's modified Eagle's medium, 10% fetal calf serum (FCS), 5 x $10^{-5}$M 2-mercaptoethanol) 5 µg/ml mycophenolic acid and 250 µg/ml xanthine (Traunecker et al., Eur. J. Immunol. 16, 851-854 [1986]). Transfection frequencies were in the range of $10^{-5}$ to $10^{-4}$.

Immunoprecipitations of the secreted proteins CD4-Mµ and CD4-Mγ2a with the appropriate antibodies or with protein A followed by Western blot analysis (Towbin et al., Proc. Natl. Acad. Sci. USA 76, 4350-4353 [1979]) showed that these proteins had the expected apparent molecular weights (Fig. 3A). The same gel electrophoresis analysis (according to U.K. Laemmli, Nature 227, 680-685 [1970]) under non-reducing conditions (by omitting 2-mercaptoethanol) revealed that CD4-Mµ was secreted as an oligomer, most likely as a pentamer, whilst CD4-Mγ2a apparently formed dimers which bound to protein A (Fig. 3, B and C).

Example 3

gp120-Binding Assay

2 x $10^7$ HTLV-IIIB-infected H9 cells (ATCC No. CRL 8543; Popovic et al., Science 224, 497-499 [1984]; Gallo et al., Science 224, 500-502 [1984]) were washed in labelling medium (cysteine-free RPMI-1640 medium supplemented with 10% fetal calf serum (FCS)), incubated for 30. min. at 37°C in 10 ml of labelling medium and then transferred to 4 ml of fresh labelling medium. After the addition of 2.5 mCi of $^{35}$S-cysteine (Amersham, UK) the incubation was continued with gentle agitation of the cells. After 4 hours the cells were transferred to fresh RPMI-1640 medium containing 10% FCS and incubated for another 14 hours. The cells were sedimented and the cell-free culture fluids of both incubation periods were mixed and cleared of virus by centrifugation at 150 000 x g at 4°C for 1 hour. 50 µl of the combined culture fluids of the labelled cells were added to 8 ml of culture fluid of the myeloma cells producing CD4-Mµ or CD4-Mγ2a and 50 µl of Sepharose-bound rat-monoclonal antibodies to either mouse IgM heavy chain or mouse kappa chain or 50 µl protein A-Sepharose (Pharmacia Uppsala, Sweden). Then the mixture was rolled overhead at 4°C for 15 hours. Thereafter the Sepharose beads were sedimented and washed in high salt and low salt washing buffer as described by Schneider et al. in Virology 132, 1-11, 1984. After the last washing the Sepharose pellets were boiled in 100 µl sample buffer. The samples were then analysed by polyacrylamide gel electrophoresis followed by fluorography to detect radiolabelled gp120 that was bound to CD4-Mµ or CD4-Mγ2a (Fig. 3,D lanes 3,5,7).

Example 4

Syncytium Assay

To assay the biological activity of the various chimaeric molecules a HIV-dependent syncytium assay, which is based on the previously reported fusigenic virus-infected cells expressing the HIV envelope gene products and uninfected adjacent cells bearing CD4 molecules [Sodroski et al., Nature 322, 470-474, (1986)] was used. A pretitrated amount of HIV-1 virus was incubated at room temperature with serially diluted recombinant proteins for 30 min. in 100µl RPMI-1640 medium. Thereafter 25 µl of each mixture was transferred in triplicate into the wells of a 96-well microtiter plate, which already contained 25,000 MT-2 cells [Myoshi, I. et al., Nature 294, 770-771 (1981)] in 50 µl medium per well. After three days of culture 100 µl of fresh medium was added and two days later syncytia were counted. 50% inhibition of syncytium formation ($ID_{50}$) was obtained with CD4-Mµ at a concentration of 10 ng/ml which was about 1000-fold less than the $ID_{50}$ (10 µg/ml) of CD4-Mγ2a and CD4-Mx. The murine soluble $L_3T_4$ receptor [Traunecker, A. et al., Immunology Today 10, 29-32 (1989)] was inactive. Complete inhibition of syncytium formation was obtained with CD4-Mµ at a concentration of 1-2 µg/ml (see Figure 4).

Example 5


Binding to Clq


Microtitration plates were coated with purified CD4-M$\gamma$2a or CD4-M$\mu$ polypeptides in saline at concentrations of 10 $\mu$g/ml (Fig.5,A) and 1 $\mu$g/ml (Fig.5,B) or as a control with mouse IgG2a or IgM at identical concentrations. After overnight incubation, the plates were blocked with 1% BSA in PBS (phosphate buffered saline) and then incubated for 6 hours at room temperature with 50.000 cpm (~2ng) $^{125}$I-labelled human Clq (Calbiochem, USA). [Radiolabelling of Clq was performed with the Iodogen method according to the recommendations of the manufacturer (Pierce, USA)]. After incubation the wells were washed and the bound radioactivity was determined. Results see Figure 5.


Example 6


Binding of CD4-M 2a to Fc-Receptors


A competition binding assay with Fc$\gamma$ receptors on murine cell line J774A.1 (ATCC No. TIB67) was used to characterize the Fc-receptor binding of CD4-M$\gamma$2a. Therefore, 2 x 10$^6$ cells were incubated with 200 ng of $^{125}$I-labelled CD4-M$\gamma$2a (radiolabelling was performed as described in Example 5) and increasing concentrations of cold competitor CD4-M$\gamma$2a or cold competitor mouse IgG2a, in 100 $\mu$l medium supplemented with 5% fetal calf serum (FCS) for 1 h at 40$^{\circ}$C. After the incubation cells were centrifuged through a 200 $\mu$l FCS cushion, and the radioactivity in the pellets was measured. The radioactivity obtained after adding a 500-fold excess of unlabelled IgG2a was taken as nonspecific binding. Specific binding was then obtained by subtracting nonspecific binding values from the experimental values.


**Claims**

1. A cloned DNA comprising a combination of two pieces of DNA whereby one of said pieces is coding for the first two domains or for parts thereof of the human CD4-molecule and the other is coding for all domains except the first of the constant regions of the heavy chain of a mammalian immunoglobulin.

2. The DNA of claim 1 wherein one of said pieces of DNA is coding for the first two domains of the human CD4-molecule.

3. The DNA of claim 1 or 2 wherein said other piece of DNA is coding for all domains except the first of the constant region of the heavy chain of a mammalian IgM.

4. The DNA of claim 1 or 2 wherein said other piece of DNA is coding for all domains except the first of the constant region of the heavy chain of a mammalian IgG.

5. The DNA of claim 1 or 2 wherein said other piece of DNA is coding for all domains except the first of the constant region of the heavy chain of a mammalian IgA.

6. The DNA of any one of claims 1 to 5 wherein the mammalian immunoglobulin is mouse or human immunoglobulin.

7. The DNA of claim 1, 2 or 4 wherein said other piece of DNA is coding for all domains except the first of the constant region of the heavy chain of a mouse Ig$\gamma$2a.

8. The DNA of claim 1, 2 or 4 wherein said other piece of DNA is coding for all domains except the first of the constant region of the heavy chain of a human Ig$\gamma$1 or Ig$\gamma$3.

9. A vector containing a DNA according to any one of claims 1-8.

10. A vector according to claim 9 which is a shuttle vector capable of replication in prokaryotic and eukaryotic host cells.

11. A vector as claimed in claim 9 or 10 which is an expression vector.

12. A prokaryotic or eukaryotic host cell transformed with a vector as claimed in anyone of claims 9 to 11.

13. A polypeptide encoded by a DNA as claimed in any one of claims 1-8.

14. The polypeptide of claim 13 as a therapeutically active agent.

15. The polypeptide of claim 13 as a therapeutically active agent against AIDS.

16. A method for the production of a polypeptide of any one of claims 13 to 15 which method comprises culturing in a suitable medium a transformed host cell as claimed in claim 12 and isolating said polypeptide.

17. A pharmaceutical composition containing at least one polypeptide as claimed in claim 13 and a therapeutically acceptable carrier material.

18. The use of a polypeptide as claimed in any one of claims 13 to 15 for the preparation of a pharmaceutical composition.

19. The use of a polypeptide as claimed in any one of claims 13 to 15 for the preparation of a pharmaceutical composition for the treatment of AIDS.

20. The polypeptide of claim 13 whenever prepared according to the method of claim 13.

Claims for the following Contracting States: GR, ES

1. A process for the production of a polypeptide encoded by a DNA comprising a combination of two pieces of DNA whereby one of said pieces is coding for the first two domains or for parts thereof of the human CD4-molecule and the other is coding for all domains except the first of the constant regions of the heavy chain of a mammalian immunoglobulin which process comprises culturing in a suitable medium a host cell transformed with a vector capable of expressing said polypeptide and isolating said polypeptide.

2. A process according to claim 1 wherein one of said pieces of DNA is coding for the first two domains of the human CD4-molecule.

3. A process according to claim 1 or 2 wherein said other piece of DNA is coding for all domains except the first of the constant region of the heavy chain of a mammalian IgM.

4. A process according to claim 1 or 2 wherein said other piece of DNA is coding for all domains except the first of the constant region of the heavy chain of a mammalian IgG.

5. A process according to claim 1 or 2 wherein said other piece of DNA is coding for all domains except the first of the constant region of the heavy chain of a mammalian IgA.

6. A process according to any one of claims 1 to 5 wherein the mammalian immunoglobulin is mouse or human immunoglobulin.

7. A process according to claim 1, 2 or 4 wherein said other piece of DNA is coding for all domains except the first of the constant region of the heavy chain of a mouse $Ig_\gamma 2a$.

8. A process according to claim 1, 2 or 4 wherein said other piece of DNA is coding for all domains except the first of the constant region of the heavy chain of a human $Ig_\gamma 1$ or $Ig_\gamma 3$.

9. A process according to any one of claims 1-8 wherein the host cell is an eukaryotic cell.

10. A process according to claim 9 wherein the host cell is a cell from a myeloma cell line.

11. A process according to claim 10 wherein the myeloma cell line is P3X63Ag8 or J558.

12. A process according to any one of claims 9-11 wherein the vector is a shuttle vector.

13. A process according to claim 10 or 11 wherein the shuttle vector is a pSV2 derived vector.

14. A process according to claim 13 wherein the pSV2 derived vector is pCD4-M$_\gamma$2a, pCD4-M$_\gamma$, pCD4-H$_\gamma$. pCD4-H$_\gamma$1 or pCD4-H$_\gamma$3.

15. A process for the preparation of a pharmaceutical composition which process comprises mixing a polypeptide obtained according to a process as claimed in any one of claims 1-14 and, if desired, one or more other therapeutically active substances with a therapeutically inert carrier material and bringing the mixture into a galenical administration form.

16. A pharmaceutical composition containing a polypeptide obtained according to a process as claimed in any one of claims 1-14 and, if desired, one or more other therapeutically active substances and a therapeutically inert carrier material.

17. The use of a polypeptide obtained according to a process as claimed in any one of claims 1-14 for the manufacture of a pharmaceutical composition.

18. The use of a polypeptide obtained according to a process as claimed in any one of claims 1-14 for the manufacture of a pharmaceutical composition for the treatment of AIDS.

Fig. 1

Fig. 2

Fig. 3

A 1 2 3 4 5 6 7

68—

43—

D ↓ ↓ ↓ ↓ ↓ ↓

gp 120 —

2 3 4 5 6 7

B 4 5 6

IgM,λ —

CD4-Mμ —

IgG2a,κ —

C 6 7 2

— IgG2a,κ

— CD4-Mγ2a

— S-L3T4

EP 0 394 827 A1

Fig. 4

y

300
200
100

x   ng ml⁻¹

1   10   100   1000   10000

Fig. 5

EP 0 394 827 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| E | EP-A-0 325 262 (THE GENERAL HOSPITAL CORP.)(26-07-1989) * Page 11, lines 7-10; pages 41-47; claims 5,6 * | 1-4,6,9 -20 | C 12 N 15/62 <br> C 12 N 15/12 <br> C 12 N 15/13 <br> C 12 N 15/85 <br> C 12 N 1/11 <br> C 12 N 5/10 <br> A 61 K 37/02 <br> C 12 P 21/00 |
| A,D | NATURE, vol. 337, 9th February 1989, pages 525-531; D.J. CAPON et al.: "Designing CD4 immunoadhesins for AIDS therapy" * The whole article * | 1-7,9- 20 | |
| P,X | NATURE, vol. 339, 4th May 1989, pages 68-70; A. TRAUNECKER et al.: "Highly efficient neutralization of HIV with recombinant CD4-immunoglobulin molecules" * The whole article * | 1-20 | |
| T | CHEMICAL ABSTRACTS, vol. 112, no. 17, 23rd April 1990, page 573, abstract no. 156570c, Columbus, Ohio, US; & US-A-344 304 (UNITED STATES DEPT. OF HEALTH AND HUMAN SERVICES) 15-08-1989 | 1-6 | TECHNICAL FIELDS SEARCHED (Int. Cl.5) <br><br> C 12 N |

The present search report has been drawn up for all claims

| Place of search | Date of completion of the search | Examiner |
|---|---|---|
| THE HAGUE | 19-07-1990 | CUPIDO M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)